# EUROPÄISCHE PATENTSCHRIFT

(11) **EP 2 629 085 B1**
(45) Veröffentlichungstag und Bekanntmachung des Hinweises auf die Patenterteilung: **03.12.2014**
(21) Anmeldenummer: 12000949.3
(22) Anmeldetag: 14.02.2012
(51) Int. Cl.: G01N 27/08, G01N 33/04

(54) **Vorrichtung zur Messung des Anteils an Käsebruch**
Device for measuring the cheese curd content
Dispositif pour mesurer la concentration de caillé

(43) Veröffentlichungstag der Anmeldung: 21.08.2013
(73) Patentinhaber: ALPMA Alpenland Maschinenbau GmbH, 83543 Rott am Inn (DE)
(72) Erfinder: Thomann, Stephan, 83022 Rosenheim (DE)
(74) Vertreter: Manitz, Finsterwald & Partner GbR

(56) Entgegenhaltungen:
- DE-A1-102009 036 633
- FR-A1- 2 273 279
- US-A- 5 987 273
- D. GLÖCKL ET AL: "Anwendungsmöglichkeiten physikalisch-chemischer Meßmethoden bei technologischen Prozessen in der Milchindustrie 2. Mitt. Thrombelastographische Untersuchungen über das Gerinnungsverhalten eingelabter Milch", NAHRUNG/FOOD, Bd. 19, Nr. 4, 1. Januar 1975 (1975-01-01) , Seiten 349-353, XP55025382, ISSN: 0027-769X, DOI: 10.1002/food.19750190407

## Beschreibung

Die Erfindung betrifft eine Messvorrichtung zur Messung des Anteils an Käsebruch in einem Bruch-Molke-Gemisch, welches in einem Behältnis aufgenommen ist, welches insbesondere als durchströmtes Rohr oder als durchströmte Wanne ausgebildet ist, wobei innerhalb eines vom Behältnis begrenzten Gemischvolumens und/oder in der Wandung des Behältnisses zumindest eine Gemischelektrode eines Sensors angeordnet ist, welcher mit einer Auswerteeinheit zur Bestimmung eines Leitfähigkeitswerts gekoppelt ist.

Während der Käseherstellung fällt in einer Zwischenstufe des Herstellungsprozesses das genannte Gemisch aus Käsebruch und Molke an. Für die Steuerung der weiteren Herstellungsschritte ist es wünschenswert, den Anteil von Käsebruch in dem Gemisch bestimmen zu können. Insbesondere soll der Anteil auch während eines Transports des Gemisches durch Rohrleitungen fortlaufend bestimmt werden können.

Da die Bestimmung des Käsebruch-Anteils messtechnisch bisher jedoch nicht befriedigend gelöst werden konnte, muss gemäß dem Stand der Technik die Menge des Bruch-Molke-Gemisches, welche einem Tank entnommen und jeweils einer Käseform zugeführt wird, während des aufeinander folgenden Befüllens mehrerer Käseformen verändert werden. Typischerweise ist nämlich beim Entnehmen von Bruch-Molke-Gemisch aus einem Tank bei einem hohen Tank-Füllstand ein größerer KäsebruchAnteil vorhanden als bei einem niedrigen Tank-Füllstand. Insofern wird mit sich reduzierendem Tank-Füllstand den Käseformen ein zunehmendes Volumen von Bruch-Molke-Gemisch zugeführt, um so sicherzustellen, dass immer und insbesondere auch bei niedrigem Tank-Füllstand ausreichend Käsebruch in eine Form gelangt. Um einen korrekten Herstellungsprozess sicherzustellen, ist es dementsprechend nötig, mit tankspezifischen, empirisch ermittelten Kurven oder Tabellen zu arbeiten, welche festlegen, welches Volumen dem Tank pro Käseform bei welchem Tank-Füllstand zu entnehmen ist.

Um diese aufwändigen Verfahren zu vereinfachen, wurde in der Vergangenheit bereits versucht, den Anteil von Käsebruch in einem Bruch-Molke-Gemisch mit einer Dichtemessung nach dem Coriolis-Prinzip oder mittels mechanischer Vorrichtungen zu ermitteln. Die Versuche lieferten jedoch keine ausreichend zuverlässigen Ergebnisse.

Die DE 10 2009 036 633 A1 beschreibt die Bestimmung eines Leitfähigkeitswerts des Bruch-Molke-Gemisches mithilfe eines Tomografiesensors. Im Rahmen einer Kalibrierung wird hierbei vorab eine Messung mit reiner Molke ohne Käsebruchanteil durchgeführt, um auf diese Weise einen Nullpunkt zu bestimmen.

Es können hier allerdings dennoch falsche Ergebnisse auftreten, wenn sich während des Abfüllens der Leitfähigkeitswert der Molke ändert. Dies geschieht beispielsweise, wenn ein Tank mit Wasser gespült wird. In diesem Fall setzt das Messsystem die verdünnte Molke, welche eine gegenüber der unverdünnten Molke erniedrigte Leitfähigkeit besitzt, irrtümlich mit einer höheren Bruchmenge gleich und reduziert das abzufüllende Volumen.

Dementsprechend besteht eine Aufgabe der vorliegenden Erfindung darin, eine verbesserte Messvorrichtung zu schaffen, mit der der Anteil von Käsebruch in einem Bruch-Molke-Gemisch mit möglichst großer Genauigkeit bestimmt werden kann.

Diese Aufgabe wird erfindungsgemäß durch die Merkmale des Patentanspruchs 1 und insbesondere dadurch gelöst, dass in einem Kalibrierungsvolumen, das im Wesentlichen lediglich Molke des Bruch-Molke-Gemisches aufweist, zumindest eine Molkeelektrode eines zweiten Sensors angeordnet ist, welcher mit der Auswerteeinheit zur wiederholten, insbesondere automatischen, Rekalibrierung der Messvorrichtung gekoppelt ist.

Unabhängig davon, an welcher Stelle die Molkeelektrode des zweiten Sensors angeordnet ist, weist das Kalibrierungsvolumen eine Separationseinheit auf, wodurch die Molke vom Bruch-Molke-Gemisch separierbar ist. Die Separationseinheit kann dabei insbesondere als ein Filter, ein Sieb und/oder ein Absaugkorb ausgebildet sein. Ein Absaugkorb ist dabei z.B. dann sinnvoll, wenn das Molkebehältnis als Rohr ausgebildet ist, dessen Einströmbereich mit dem Tank oder dem Koagulator verbunden ist.

Erfindungsgemäß wird somit sowohl die Leitfähigkeit des Bruch-Molke-Gemisches, als auch insbesondere der Leitfähigkeitswert der Molke gemessen. Die Rekalibrierung der Messvorrichtung, also insbesondere die Korrektur des Leitfähigkeitswerts des Bruch-Molke-Gemisches, erfolgt hier nicht lediglich einmalig zu Beginn, sondern mehrmals während des insbesondere kontinuierlichen Messvorgangs. Eine Kalibrierung kann beispielsweise durchgeführt werden, indem die Leitfähigkeit des Bruch-Molke-Gemisches auf die Leitfähigkeit der Molke normiert wird.

Auf diese Weise werden Änderungen, wie beispielsweise der Dichte, der Konzentration oder der Zusammensetzung der Molke, bei der Messung des Leitfähigkeitswerts des Bruch-Molke-Gemisches kompensiert, d.h. der Anteil des Käsebruchs wird im Wesentlichen unabhängig von den Eigenschaften der Molke bestimmt.

Der Anteil an Käsebruch in einem Bruch-Molke-Gemisch ist somit messtechnisch zu jedem Zeitpunkt während der Käseherstellung derart genau bestimmbar, dass das Volumen eines in eine Käseform eingefüllten Bruch-Molke-Gemisches so dosiert werden kann, dass der Käseform immer die exakt benötigte Menge an Käsebruch zugeführt wird. Die Verwendung der vorstehend erwähnten tankspezifischen Kurven bzw. Tabellen und der Einsatz der damit verbundenen aufwändigen Steuerprozesse kann somit ersatzlos eingespart werden. Erfindungsgemäß kann die Steuerung der einer Käseform zuzuführenden Menge an Käsebruch durch die Steuerung der pro Zeiteinheit fließenden Bruch-Molke-Gemisch-Menge (Durchfluss) und/oder durch Steuerung der Zeit, die für einen Befüllvorgang zur Verfügung steht (Abfülltakt) bewirkt werden. Alternativ ist es möglich, aus einem Tank mit Bruch-Molke-Gemisch immer genau so viel Molke abzuziehen, dass die Bruch-Konzentration im Tank konstant bleibt. In diesem Fall ist es dann nicht unbedingt nötig, den Durchfluss und den Abfülltakt, mit dem die Käseformen befüllt werden, zu variieren, da die Menge von pro Zeiteinheit der Käseform zugeführtem Bruch bei konstantem Durchfluss aufgrund der konstanten Bruch-Konzentration im Tank ebenfalls immer konstant ist.

Der erfindungsgemäß über den ersten Sensor und dessen Gemischelektrode ermittelte Leitfähigkeitswert, bei dem es sich insbesondere um einen mittleren Leitfähigkeitswert handelt, gibt somit ausreichend genau Aufschluss darüber, wie hoch der Anteil an Käsebruch in einem Bruch-Molke-Gemisch ist. Dies wird grundsätzlich durch die Tatsache ermöglicht, dass Käsebruch eine andere Leitfähigkeit besitzt als Molke. Einer Veränderung der Molke wird dabei durch die mit Hilfe des zweiten Sensors und dessen Molkeelektrode erfolgende Rekalibrierung Rechnung getragen, wodurch verfälschte Messungen vermieden werden. Somit wird es erfindungsgemäß erstmals möglich, den tatsächlichen Anteil an Käsebruch in einem Bruch-Molke-Gemisch unabhängig von den Eigenschaften der Molke zu bestimmen.

Ein weiterer Vorteil der Erfindung besteht darin, dass ein gemäß Stand der Technik oftmals eingesetzter Entmolkungstank, welcher zwischen einen das Bruch-Molke-Gemisch bereitstellenden Puffertank und die zu befüllenden Käseformen geschaltet ist, ebenfalls vollständig eingespart werden kann. Ein derartiger Entmolkungstank wurde gemäß Stand der Technik eingesetzt, um in Abhängigkeit von einer puffertankspezifischen Kurve mit Bruch-Molke-Gemisch derart befüllt zu werden, dass sich im Entmolkungstank immer ein konstantes Volumen und ein konstanter Käsebruch-Anteil befindet, sodass dem Entmolkungstank pro Käseform immer das gleiche Volumen entnommen werden konnte. Dies ist erfindungsgemäß nicht mehr nötig, da dem Puffertank aufgrund des Einsatzes der Sensoren immer genau diejenige Menge an Bruch-Molke-Gemisch direkt entnommen werden kann, die pro Käseform benötigt wird. Alternativ betrifft die Erfindung jedoch auch solche Anordnungen, die weiterhin mit einem Entmolkungstank ausgestattet sind.

Weitere bevorzugte Ausgestaltungen der Erfindung sind in den abhängigen Ansprüchen, der Beschreibung sowie den Zeichnungen angegeben.

Gemäß einer Ausführungsform ist das Kalibrierungsvolumen in einem separaten Molkebehältnis, welches insbesondere als durchströmtes Rohr oder als durchströmte Wanne ausgebildet ist, angeordnet. Das Gemischvolumen und das Kalibrierungsvolumen sind somit örtlich voneinander getrennt.

Nach einer weiteren Ausführungsform sind sowohl das Behältnis, welches das Bruch-Molke-Gemisch aufnimmt, als auch das Molkebehältnis durch ein einziges durchströmtes Rohr oder eine einzige durchströmte Wanne gebildet. In diesem Fall ist von Vorteil, dass die Gemischelektrode und die Molkeelektrode räumlich nahe beieinander angeordnet werden können, um so möglichst unverfälschte Messergebnisse zu erhalten. Idealerweise befinden sich die Gemischelektrode und die Molkeelektrode an ein und derselben Stelle, in der Praxis wird ein Abstand von wenigen Zentimetern angestrebt. Abstände zwischen 1 cm und 50 cm erweisen sich als sinnvoll, wobei es, wie bereits erwähnt, ein Ziel ist, diesen Abstand möglichst klein zu halten.

Ein durchströmtes Rohr, in welchem sich die Gemischelektrode und die Molkeelektrode befinden, kann dabei beispielsweise einen Rohrdurchmesser von ungefähr 2,5 Inch haben.

Es ist jedoch auch denkbar, dass das Behältnis, in dem sich die Gemischelektrode und/oder die Molkeelektrode befinden, nicht als Rohr, sondern als Tank oder Koagulator für das Bruch-Molke-Gemisch auszubilden.

In einer weitergebildeten Ausführungsform sind die Gemischelektroden und/oder die Molkeelektroden in oder an der Wandung des Rohres oder der Wanne und bevorzugt entlang einer Mantellinie des Rohres oder der Wanne insbesondere gleich verteilt angeordnet. Durch eine solche Anordnung lassen sich besonders zuverlässige Messergebnisse erzielen.

Es ist bevorzugt, wenn die Gemischelektroden und/oder die Molkeelektroden bündig mit der Innenwand des Rohres oder der Wanne abschließen, da sie in diesem Fall die Strömung des Bruch-Molke-Gemisches in keiner Weise beeinflussen oder behindern. Ein bündiges Abschließend der Elektroden mit der Innenwand des Rohres oder der Wanne ist ferner unter hygienischen Gesichtspunkten vorteilhaft, da eine Reinigung des Rohres bzw. der Wanne in diesem Fall problemlos möglich ist.

Gemäß einer weiteren Ausführungsform sind das Behältnis, welches das Bruch-Molke-Gemisch aufnimmt, und das Molkebehältnis als separate Rohre oder Wannen ausgebildet, deren Einströmbereiche mit einem Tank und/oder einem Koagulator für das Bruch-Molke-Gemisch verbunden sind. Die Einströmbereiche des Behältnisses und des Molkebehältnisses können dabei insbesondere über ein gemeinsames Teilstück verfügen, das mit dem Tank oder dem Koagulator verbunden ist. Es ist jedoch auch denkbar, dass sowohl das Behältnis als auch das Molkebehältnis über einen separaten Anschluss an den Tank oder den Koagulator verfügen.

Nach einer weiteren Ausführungsform sind bzw. ist der Ausströmbereich des Behältnisses und/oder der Ausströmbereich des Molkebehältnisses mit dem Tank und/oder dem Koagulator für das Bruch-Molke-Gemisch verbunden. Insbesondere sind dabei sowohl das Behältnis als auch das Molkebehältnis als Rohre ausgebildet, welche wie ein Bypass aus einem Tank oder Koagulator führen und anschließend wieder in den Tank oder Koagulator zurückführen. Das zu messende Bruch-Molke-Gemisch bzw. die zu messende Molke strömt somit an einer Stelle des Tanks oder Koagulators in ein Rohr, in dem eine Elektrode zur Messung des Leitfähigkeitswerts angeordnet ist, und anschließend wieder in den Tank oder Koagulator zurück.

In einer alternativen Ausführungsform sind bzw. ist der Ausströmbereich des Behältnisses und/oder der Ausströmbereich des Molkebehältnisses mit einer Förderleitung verbunden. Hierbei wird das Bruch-Molke-Gemisch bzw. die Molke also nicht wieder zurück in den Tank und/oder den Koagulator geleitet, sondern fließt direkt, insbesondere über eine gemeinsame Förderleitung, zu einer Abfülleinheit zur Einbringung des Bruch-Molke-Gemisches in Käseformen.

Gemäß einer weiteren alternativen Ausführungsform der Erfindung sind nur der Einströmbereich und der Ausströmbereich des Molkebehältnisses mit dem insbesondere als Tank oder Koagulator ausgebildeten Behältnis des Bruch-Molke-Gemisches verbunden. Die Leitfähigkeit des Bruch-Molke-Gemisches wird hierbei also insbesondere direkt im Tank oder Koagulator gemessen, während das beispielsweise als Rohr ausgebildete Molkebehältnis als Bypass zunächst aus dem Tank oder Koagulator hinaus und schließlich wieder hinein führt.

Ebenso ist es denkbar, dass auch das Kalibrierungsvolumen innerhalb des insbesondere als Tank oder Koagulator ausgebildeten Behältnisses angeordnet ist. Es sind hierbei insbesondere die Elektroden beider Sensoren innerhalb des mit Bruch-Molke-Gemisch gefüllten Behältnisses angeordnet. Der Bereich, in dem sich die Molkeelektrode des zweiten Sensor befindet, wird hierbei beispielsweise mithilfe von Filtern gegenüber den Käsebruch-Bestandteilen des Gemisches abgeschirmt, damit sich in diesem Bereich im Wesentlichen lediglich Molke befindet.

Gemäß einer vorteilhaften Ausführungsform weist die Separationseinheit ein Sieb, beispielsweise ein Spaltrohrsieb oder ein Lochblechsieb, auf. Vorzugsweise wird ein Spaltrohrsieb mit einer Spaltgröße von 0,15 mm und 10 % offener Fläche eingesetzt. Dieses wird in Strömungsrichtung angeordnet, d.h. der Winkel, den die Fläche des Siebs mit der Strömungsrichtung einschließt, beträgt maximal 45°. Dadurch wird eine große Filterfläche, beispielsweise 50 x 450mm, zur Verfügung gestellt. Somit ist ein kontinuierliches Abführen der Molke möglich, beispielsweise bei einem Volumenstrom in der Hauptleitung von 50001/h, ohne dass es zu einem Abreißen der Strömung kommt. Um zudem ein Verblocken der Filter möglichst gering zu halten, wird vorzugsweise zunächst der Tank und/oder Koagulator befüllt, bevor der Bypass für das Kalibrierungsvolumen beispielsweise über ein Ventil geöffnet wird.

Gemäß einer weiteren Ausführungsform ist die Auswerteeinheit derart ausgebildet, dass die Rekalibrierung zeitverzögert, beispielsweise nach 1,5 bis 2 Minuten, erfolgt, was bedeutet, dass eine Rekalibrierung z.B. erst bei solchen Messungen des ersten Sensors berücksichtigt wird, die 1,5 bis 2 Minuten nach der Rekalibrierung erfolgen.

Beim Einsatz der erfindungsgemäßen Vorrichtung ist es zur Erzielung der erforderlichen Genauigkeit wünschenswert, die Leitfähigkeitsmessungen mittels des ersten Sensors relativ häufig durchzuführen. Eine Messfrequenz von 20 Messungen pro Sekunde kann dabei durchaus zum Einsatz gelangen.

Gemäß einer weiteren Ausführungsform ist zumindest einer der Sensoren als Tomographiesensor ausgebildet. Besonders gute Ergebnisse lassen sich dann erzielen, wenn als Tomographiesensor ein ERT-Sensor eingesetzt wird (ERT = Electrical Resistance Tomography).

Vorteilhaft ist es, wenn zumindest einer der Sensoren zusätzlich zur Temperatur- und/oder zur Druck- und/oder zur Volumenstrommessung ausgebildet ist. Die auf diese Weise ermittelten zusätzlichen Messwerte können ebenfalls zu einer vorteilhaften Steuerung eines Käseherstellungsprozesses eingesetzt werden. Da die erfindungsgemäßen Sensoren insbesondere für bestimmte zulässige Temperaturbereiche kalibriert werden können, ist es bei zusätzlicher Ermittlung von Temperaturwerten durch den Sensor möglich, ein Warnsignal abzugeben, wenn die Temperatur des Bruch-Molke-Gemisches einen vorgegebenen, zulässigen Temperaturbereich verlässt. Alternativ ist es möglich, für unterschiedliche Temperaturbereiche unterschiedliche Kalibrierungsparameter des Sensors zu hinterlegen und diese dann in Abhängigkeit von den vom Sensor ermittelten Gemisch-Temperaturen zu aktivieren. Beispielsweise ist es möglich, die vom Sensor ermittelten Leitfähigkeitswerte um ungefähr 2 % pro 1°C Temperaturänderung zu korrigieren. So kann eine automatische, temperaturabhängige Kompensation von Messfehlern erfolgen, die sich durch Temperaturänderungen im Bruch-Molke-Gemisch oder in der Molke ergeben könnten. Besonders sinnvoll ist es, wenn die Messungen in einem durchströmten Rohr oder in einer durchströmten Wanne in Abhängigkeit vom Volumenstrom ausgeführt werden. Hier ist beispielsweise denkbar, eine Messung immer dann auszulösen, wenn in einem durchströmten Rohr ein Volumen von 0,1 1 seit der letzten Messung am Sensor vorbeigeströmt ist. Bei einer solchen Variante ist es besonders vorteilhaft, wenn der Sensor selbst zusätzlich auch zur Volumenstrommessung ausgelegt ist, da dann Leitfähigkeitsmessungen durch den Sensor aufgrund von Volumenstrommesswerten ausgelöst werden können, die vom Sensor selbst ermittelt wurden.

Gemäß einer weiteren Ausführungsform sind der erste und der zweite Sensor als ein gemeinsamer Sensor ausgebildet, dessen zumindest eine Gemischelektrode innerhalb des und/oder angrenzend an das Gemischvolumen und dessen zumindest eine Molkeelektrode innerhalb des und/oder angrenzend an das Kalibrierungsvolumen angeordnet ist. Auf diese Weise wird nur ein einziger Sensor eingesetzt, der über Elektroden im Bruch-Molke-Gemisch und in der Molke sowohl die Leitfähigkeit des Bruch-Molke-Gemisches als auch die Leitfähigkeit der Molke misst. Durch den Einsatz nur eines einzelnen Sensors können Kosten gespart werden.

Die Erfindung wird nachfolgend anhand von Ausführungsbeispielen unter Bezugnahme auf die Zeichnungen beschrieben. In diesen zeigen:
- Fig. 1: ein Blockschaltbild einer erfindungsgemäßen Messvorrichtung mit Elektroden in zwei Bypässen,
- Fig. 2: ein Blockschaltbild der Messvorrichtung gemäß Fig. 1 mit einem gemeinsamen Einströmbereich der Bypässe,
- Fig. 3: ein Blockschaltbild einer erfindungsgemäßen Messvorrichtung mit Elektroden in zwei Förderleitungen,
- Fig. 4: ein Blockschaltbild der Messvorrichtung gemäß Fig. 3 mit einem gemeinsamen Einströmbereich der Förderleitungen,

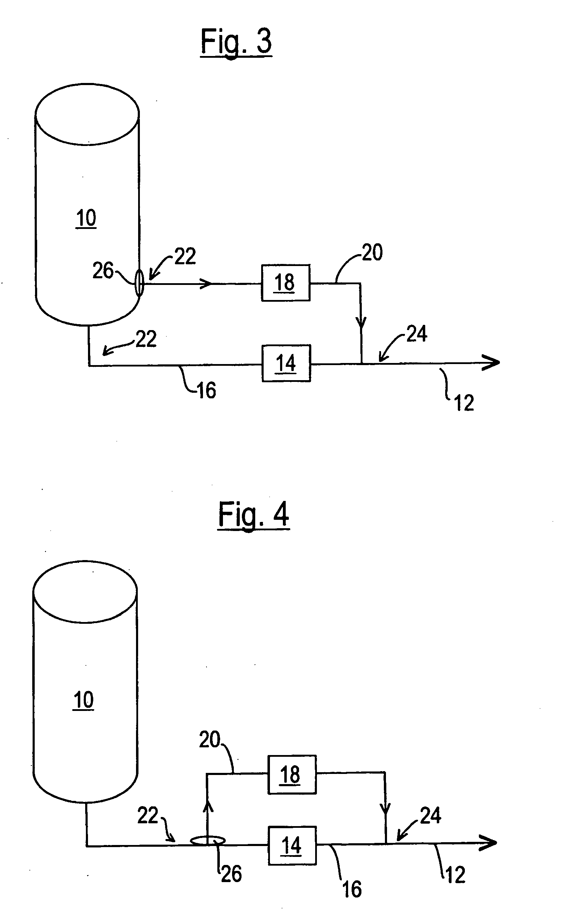

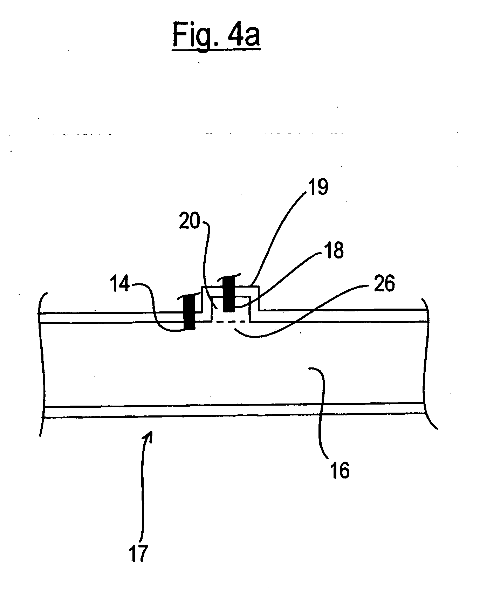

- Fig. 4 a: einen schematischen Querschnitt durch ein Rohr, in dem das Gemisch- und das Kalibrierungsvolumen vorgesehen sind,

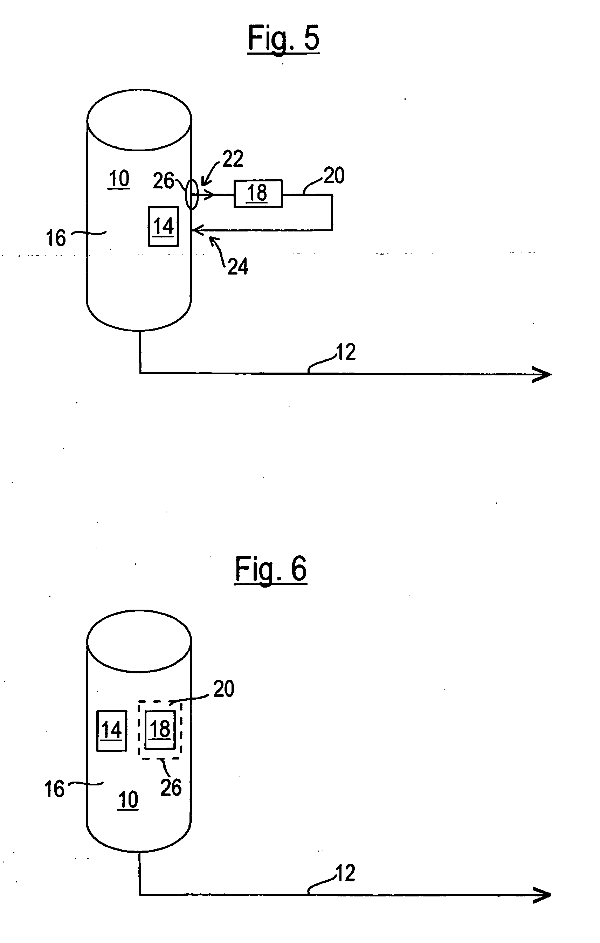

- Fig. 5: ein Blockschaltbild einer erfindungsgemäßen Messvorrichtung mit einer Elektrode in einem Tank und einer Elektrode in einem Bypass, und
- Fig. 6: ein Blockschaltbild einer erfindungsgemäßen Messvorrichtung mit zwei Elektroden in einem Tank.

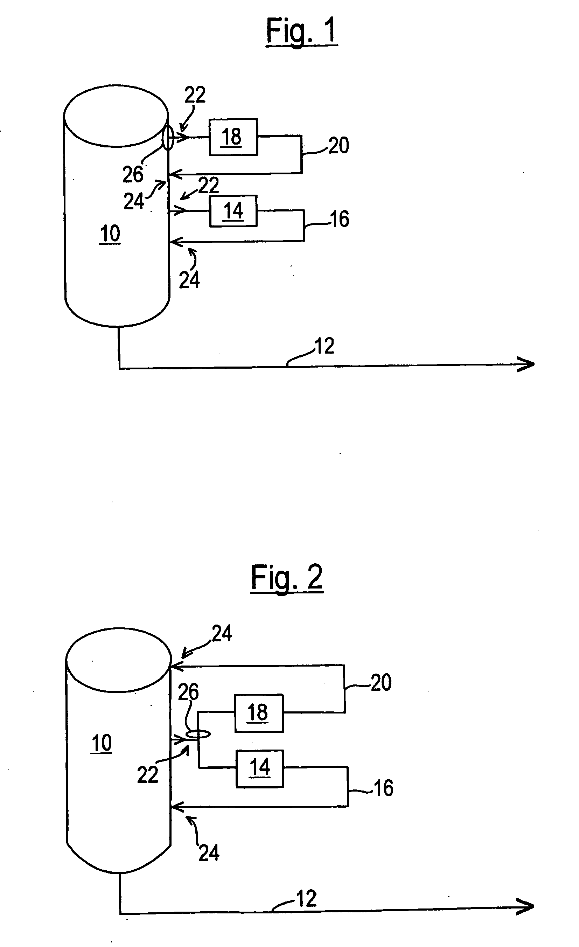

Fig. 1 bis Fig. 6 zeigen jeweils in schematischer Ansicht einen Tank 10, der mit einem Bruch-Molke-Gemisch gefüllt ist. Aus diesem Tank 10 führt eine Förderleitung 12 zu einer nicht dargestellten Abfülleinheit zur Einbringung des Bruch-Molke-Gemisches in Käseformen.

Zumindest eine Gemischelektrode 14 eines nicht dargestellten ersten Sensors ist hierbei in einem Gemischvolumen 16, in welchem sich Bruch-Molke-Gemisch befindet, angeordnet. Zudem befindet sich zumindest eine Molkeelektrode 18 eines nicht dargestellten zweiten Sensors, welcher mit dem ersten Sensor als ein gemeinsamer Sensor ausgebildet sein kann, in einem Kalibrierungsvolumen 20, in dem sich im Wesentlichen ausschließlich Molke befindet. Zur Bestimmung eines Leitfähigkeitswerts sind der erste Sensor und der zweite Sensor mit einer nicht dargestellten Auswerteeinheit verbunden.

In der Ausführungsform gemäß Fig. 1 befindet sich das Gemischvolumen 16 in einem Behältnis 16, welches als durchströmtes Rohr 16 ausgebildet ist. In diesem Rohr 16 befinden sich die Gemischelektroden 14 des ersten Sensors. Das Rohr 16 beginnt an seinem Einströmbereich 22 am Tank 10 und endet an seinem Ausströmbereich 24 ebenfalls am Tank 10. Es bildet somit einen Bypass zum Tank 10.

Das Kalibrierungsvolumen 20 befindet sich in einem Molkebehältnis 20, welches als ein weiteres durchströmtes Rohr 20 ausgebildet ist. In diesem Rohr 20 sind die Molkeelektroden 18 des zweiten Sensors angeordnet. Das Rohr 20 ist an seinem Einströmbereich 22 mit dem Tank 10 verbunden und endet an seinem Ausströmbereich 24 wieder am Tank 10. Auch das als Molkebehältnis 20 dienende Rohr 20 bildet somit einen Bypass zum Tank 10. Um lediglich Molke in das Rohr 20 einströmen zu lassen, ist im Einströmbereich 22 ein Filter 26, insbesondere ein Spaltrohrsieb, als Separationseinheit 26 angeordnet. Das Filter 26 ist beispielsweise als Absaugkorb ausgebildet.

Um das Bruch-Molke-Gemisch bzw. die Molke in die Rohre 16, 20 zu befördern, sind beispielsweise nicht dargestellte Pumpen vorgesehen.

Fig. 2 zeigt eine alternative Ausführungsform der Erfindung. Auch hier sind sowohl die Gemischelektroden 14 des ersten Sensors als auch die Molkeelektroden 18 des zweiten Sensors in einem Bypass zum Tank 10 angeordnet. Die Rohre 16, 20 haben hier jedoch im Einströmbereich 22 ein gemeinsames Teilstück. Die Rohre 16, 20 enden dagegen wieder an separaten Stellen im Tank.

Im Ausführungsbeispiel gemäß Fig. 3 und Fig. 4 sind die Elektroden 14, 18 in der Förderleitung 12 angeordnet. Gemäß Fig. 3 haben das Behältnis 16 und das Molkebehältnis 20, die jeweils als durchströmte Rohre 16, 20 ausgebildet sind, separate Einströmbereiche 22, die jeweils an unterschiedlichen Stellen mit dem Tank 10 verbunden sind. Gemäß Fig. 4 verfügen die Rohre 16, 20 dagegen über einen gemeinsamen Einströmbereich 22. In diesem Fall führt also nur eine einzige Leitung aus dem Tank 10.

Fig. 4a zeigt eine besonders bevorzugte Ausführungsform der Erfindung, bei welcher das Behältnis für das Bruch-Molke-Gemisch und das Molkebehältnis als ein einziges durchströmtes Rohr 17 ausgebildet sind. In diesem durchströmten Rohr 17 befindet sich somit sowohl das Gemischvolumen 16 als auch das Kalibrierungsvolumen 20. Das Rohr 17 weist eine Querschnittserweiterung 19 auf, innerhalb welcher sich das Kalibrierungsvolumen 20 befindet. Kalibrierungsvolumen 20 und Gemischvolumen 16 sind durch ein Filter bzw. Sieb 26 voneinander getrennt, um so sicherzustellen, dass kein Käsebruch in das Kalibrierungsvolumen 20 gelangt.

Innerhalb des Kalibrierungsvolumens 20 befindet sich die Molkeelektrode 18, wohingegen sich die Gemischelektrode 14 innerhalb des Gemischvolumens 16 befindet. Die beiden Elektroden 14, 18 sind gemäß Fig. 4a räumlich sehr nahe beieinander angeordnet, um so möglichst unverfälschte Messergebnisse zu ermöglichen.

Gemäß Fig. 5 befindet sich das Gemischvolumen 16 im Tank 10. Die Gemischelektroden 14 des ersten Sensor sind somit ebenfalls direkt im Tank 10 angeordnet. Die Leitfähigkeit der Molke wird - entsprechend Fig. 1 - mithilfe eines Bypasses gemessen.

Gemäß Fig. 6 sind nicht nur die Gemischelektroden 14 des ersten Sensors direkt im Tank 10 angeordnet, sondern auch die Molkeelektroden 18 des zweiten Sensors. Um im Tank 10 ein Kalibrierungsvolumen 20 zu erhalten, in dem sich lediglich Molke befindet, sind die Molkeelektroden 18 insbesondere allseitig von einem Filter 26 umgeben, der in Fig. 6 als gestrichelte Linie dargestellt ist.

Unabhängig von der Anordnung der Elektroden 14, 18 wird über den ersten Sensor permanent die Leitfähigkeit des Bruch-Molke-Gemisches gemessen. Zudem misst der zweite Sensor stets die Leitfähigkeit der Molke. Mit diesen Daten wird in der Auswerteeinheit der Leitfähigkeitswert des Bruch-Molke-Gemisches korrigiert, um so etwaigen Änderungen der Molke, die sich auf deren Leitfähigkeit auswirken, Rechnung zu tragen. Es findet somit eine wiederholte, bevorzugt stete und insbesondere automatische Rekalibrierung der Messvorrichtung statt.

Auf diese Weise kann der Anteil an Käsebruch im Bruch-Molke-Gemisch zu jedem Zeitpunkt zuverlässig bestimmt werden. Beim Betrieb einer Vorrichtung gemäß Fig. 1 bis Fig. 6 soll sichergestellt werden, dass jeder Käseform immer die gleiche Menge an Käsebruch zugeführt wird. Zu diesem Zweck wird auf Grundlage des ermittelten Käsebruch-Anteils eine dem Tank 10 zugeordnete, nicht dargestellte Pumpe, die dazu dient, Molke aus dem Tank 10 beispielsweise in einen Puffertank abzuziehen, entsprechend angesteuert. Wenn beispielsweise ein zu hoher Anteil an Käsebruch festgestellt wird, muss die Leistung der Pumpe verringert werden, sodass dem Tank 10 weniger Molke entzogen und dadurch der Anteil an Käsebruch im Gemisch erniedrigt wird. Auf der anderen Seite wird die Leistung der Pumpe erhöht, wenn über die Sensoren ein zu geringer Anteil an Käsebruch festgestellt wird. Letztlich soll die Pumpe in Abhängigkeit von über die Sensoren ermittelten Messdaten so angesteuert werden, dass der Anteil an Käsebruch im Tank 10 immer im Wesentlichen konstant ist. In diesem Fall kann dann die nicht dargestellte Pumpe in der Förderleitung 12 immer mit konstantem Abfülltakt und konstanter Leistung betrieben werden, da der Anteil an Käsebruch im Tank 10 bereitgestellten Bruch-Molke-Gemisch ebenfalls immer konstant ist.

Alternativ kann unter Einsparung des genannten Puffertanks auch eine Steuerung der nicht dargestellte Pumpe in der Förderleitung 12 in Abhängigkeit von dem erfindungsgemäß ermittelten Anteil an Käsebruch im Bruch-Molke-Gemisch erfolgen. Bei einem niedrigeren Anteil wird die Pumpe dann mit entsprechend erhöhter Leistung und bei einem höheren Anteil mit entsprechend erniedrigter Leistung betrieben.

### Bezugszeichenliste

- 10: Tank
- 12: Förderleitung
- 14: Gemischelektrode
- 16: Gemischvolumen, Behältnis, Rohr
- 17: Rohr
- 18: Molkeelektrode
- 19: Querschnittserweiterung
- 20: Kalibrierungsvolumen, Molkebehältnis, Rohr
- 22: Einströmbereich
- 24: Ausströmbereich
- 26: Filter, Separationseinheit

## Patentansprüche

1. Messvorrichtung zur Messung des Anteils an Käsebruch in einem Bruch-Molke-Gemisch, welches in einem Behältnis (16) aufgenommen ist und welches insbesondere als durchströmtes Rohr oder als durchströmte Wanne ausgebildet ist, wobei innerhalb eines vom Behältnis (16) begrenzten Gemischvolumens und/oder in der Wandung des Behältnisses (16) zumindest eine Gemischelektrode (14) eines ersten Sensors angeordnet ist, welcher mit einer Auswerteeinheit zur Bestimmung eines Leitfähigkeitswerts gekoppelt ist,
**dadurch gekennzeichnet, dass**
in einem Kalibrierungsvolumen (20), das im Wesentlichen lediglich Molke des Bruch-Molke-Gemisches aufweist, zumindest eine Molkeelektrode (18) eines zweiten Sensors angeordnet ist, welcher mit der Auswerteeinheit zur wiederholten Rekalibrierung der Messvorrichtung gekoppelt ist, wobei das Kalibrierungsvolumen (20) eine Separationseinheit, insbesondere ein Filter (26), ein Sieb und/oder einen Absaugkorb, aufweist, wodurch die Molke vom Bruch-Molke-Gemisch separierbar ist.

2. Messvorrichtung nach Anspruch 1,
**dadurch gekennzeichnet, dass**
das Kalibrierungsvolumen (20) in einem separaten Molkebehältnis (20), welches insbesondere als durchströmtes Rohr oder als durchströmte Wanne ausgebildet ist, angeordnet ist.

3. Messvorrichtung nach Anspruch 1,
**dadurch gekennzeichnet , dass**
das sowohl das Behältnis (16) als auch das Molkebehältnis (20) durch ein einziges durchströmtes Rohr (17) oder eine einzige durchströmte Wanne gebildet sind.

4. Messvorrichtung nach einem der vorhergehenden Ansprüche,
**dadurch gekennzeichnet , dass**
die Gemischelektroden (14) und/oder die Molkeelektroden (18) in oder an der Wandung des Rohres (16, 17, 20) oder der Wanne und bevorzugt entlang einer Mantellinie des Rohres (16, 20) oder der Wanne insbesondere gleichverteilt angeordnet sind.

5. Messvorrichtung nach einem der vorhergehenden Ansprüche,
**dadurch gekennzeichnet , dass**
die Gemischelektroden (14) und/oder die Molkeelektroden (18) bündig mit der Innenwand des Rohres (16, 17, 20) oder der Wanne abschließen.

6. Messvorrichtung nach zumindest einem der vorhergehenden Ansprüche,
**dadurch gekennzeichnet , dass**
das Behältnis (16) und das Molkebehältnis (20) als separate Rohre oder Wannen ausgebildet sind, deren Einströmbereiche (22), insbesondere über ein gemeinsames Teilstück, mit einem Tank (10) und/oder einem Koagulator für das Bruch-Molke-Gemisch verbunden sind.

7. Messvorrichtung nach Anspruch 6,
**dadurch gekennzeichnet , dass**
der Ausströmbereich (24) des Behältnisses (16) und/oder der Ausströmbereich (24) des Molkebehältnisses (20) mit dem Tank (10) und/oder dem Koagulator für das Bruch-Molke-Gemisch verbunden sind/ist.

8. Messvorrichtung nach Anspruch 6,
**dadurch gekennzeichnet , dass**
der Ausströmbereich (24) des Behältnisses (16) und/oder der Ausströmbereich (24) des Molkebehältnisses (20), insbesondere über ein gemeinsames Teilstück, mit einer Förderleitung (12) verbunden sind/ist, die insbesondere zu einer Abfülleinheit zur Einbringung des Bruch-Molke-Gemisches in Käseformen führt.

9. Messvorrichtung nach zumindest einem der Ansprüche 2 bis 5,
**dadurch gekennzeichnet , dass**
der Einströmbereich (22) und der Ausströmbereich (24) des Molkebehältnisses (20) mit dem, insbesondere als Tank (10) oder Koagulator ausgebildeten Behältnis (16) des Bruch-Molke-Gemisches verbunden sind.

10. Messvorrichtung nach einem der vorhergehenden Ansprüche,
**dadurch gekennzeichnet , dass**
das Kalibrierungsvolumen (20) innerhalb des, insbesondere als Tank (10) oder Koagulator ausgebildeten Behältnisses (16) des Bruch-Molke-Gemisches angeordnet ist.

11. Messvorrichtung nach einem der vorhergehenden Ansprüche,
**dadurch gekennzeichnet, dass**
die Separationseinheit (26) ein Sieb, insbesondere ein Spaltrohrsieb, aufweist, dessen Fläche einen Winkel von maximal 45° mit der Strömungsrichtung einschließt.

12. Messvorrichtung nach zumindest einem der vorhergehenden Ansprüche,
**dadurch gekennzeichnet, dass**
die Auswerteeinheit derart ausgebildet ist, dass die Rekalibrierung zeitverzögert, insbesondere bei Erreichen eines Gleichgewichts im Gemischvolumen (16, 17) und/oder Kalibrierungsvolumen (20, 17), erfolgt.

13. Messvorrichtung nach zumindest einem der vorhergehenden Ansprüche,
**dadurch gekennzeichnet, dass**
zumindest einer der Sensoren als Tomografiesensor, insbesondere als ERT-Sensor ausgebildet ist.

14. Messvorrichtung nach zumindest einem der vorhergehenden Ansprüche,
**dadurch gekennzeichnet, dass**
zumindest einer der Sensoren zusätzlich zur Temperatur- und/oder zur Druck- und/oder zur Volumenstrommessung ausgebildet ist.

15. Messvorrichtung nach zumindest einem der vorhergehenden Ansprüche,
**dadurch gekennzeichnet , dass**
der erste und der zweite Sensor als ein gemeinsamer Sensor ausgebildet sind, dessen zumindest eine Gemischelektrode (14) innerhalb des und/oder angrenzend an das Gemischvolumen (16, 17) und dessen zumindest eine Molkeelektrode (18) innerhalb des und/oder angrenzend an das Kalibrierungsvolumen (20, 17) angeordnet ist.

## Claims

1. A measuring apparatus for measuring the proportion of curds in a mixture of curds and whey, the mixture being received in a container (16) and the container in particular being configured as a pipe which is flowed through or as a tub which is flowed through, wherein at least one mixture electrode (14) of a first sensor is arranged within a mixture volume bounded by the container (16) and/or in the wall of the container (16), the sensor being coupled to an evaluation unit for determining a conductivity value,
**characterised in that**
at least one whey electrode (18) of a second sensor, which is coupled to the evaluation unit for a repeated recalibration of the measuring apparatus, is arranged in a calibration volume (20) which substantially has only whey of the mixture of curds and whey, wherein the calibration volume (20) has a separation unit, in particular a filter (26), a screen and/or a suction basket, whereby the whey can be separated from the mixture of curds and whey

2. A measuring apparatus in accordance with claim 1,
**characterised in that**
the calibration volume (20) is arranged in a separate whey container (20) which is in particular configured as a pipe which is flowed through or as a tub which is flowed through.

3. A measuring apparatus in accordance with claim 1,
**characterised in that**
both the container (16) and the whey container (20) are formed a by a single pipe (17) which is flowed through or by a single tub which is flowed through.

4. A measuring apparatus in accordance with any one of the preceding claims,
**characterised in that**
the mixture electrodes (14) and/or the whey electrodes (18) are arranged, in particular equally distributed, in or at the wall of the pipe (16, 17, 20) or of the tub and preferably along a surface line of the pipe (16, 20) or of the tub.

5. A measuring apparatus in accordance with any one of the preceding claims,
**characterised in that**
the mixture electrodes (14) and/or the whey electrodes (18) terminate flush with the inner wall of the pipe (16, 17, 20) or of the tub.

6. A measuring apparatus in accordance with at least one of the preceding claims,
**characterised in that**
the container (16) and the whey container (20) are configured as separate pipes or tubs whose inflow zones (22) are connected, in particular via a common part piece, to a tank (10) and/or to a coagulator for the mixture of curds and whey.

7. A measuring apparatus in accordance with claim 6,
**characterised in that**
the outflow zone (24) of the container (16) and/or the outflow zone (24) of the whey container (20) is/are connected to the tank (10) and/or to the coagulator for the mixture of curds and whey.

8. A measuring apparatus in accordance with claim 6,
**characterised in that**
the outflow zone (24) of the container (16) and/or the outflow zone (24) of the whey container (20) is/are connected, in particular via a common part piece, to a conveying line (12) which in particular leads to a filling unit for introducing the mixture of curds and whey into cheese moulds.

9. A measuring apparatus in accordance with at least one of the claims 2 to 5,
**characterised in that**
the inflow zone (22) and the outflow zone (24) of the whey container (20) are connected to the container (16), in particular configured as a tank (10) or coagulator, of the mixture of curds and whey.

10. A measuring apparatus in accordance with any one of the preceding claims,
**characterised in that**
the calibration volume (20) is arranged within the container (16), in particular configured as a tank (10) or as a coagulator, of the mixture of curds and whey.

11. A measuring apparatus in accordance with any one of the preceding claims,
**characterised in that**
the separation unit (26) has a screen, in particular a slot strainer, whose surface includes an angle of a maximum of 45° with the flow direction.

12. A measuring apparatus in accordance with at least one of the preceding claims,
**characterised in that**
the evaluation unit is configured such that the recalibration takes place with a time delay, in particular on reaching an equilibrium in the mixed volume (16, 17) and/or in the calibration volume (20, 17).

13. A measuring apparatus in accordance with at least one of the preceding claims,
**characterised in that**
at least one of the sensors is configured as a tomography sensor, in particular as an ERT sensor.

14. A measuring apparatus in accordance with at least one of the preceding claims,
**characterised in that**
at least one of the sensors is additionally configured for the measurement of temperature, pressure and/or volume.

15. A measuring apparatus in accordance with at least one of the preceding claims,
**characterised in that**
the first and second sensors are configured as a common sensor whose at least one mixture electrode (14) is arranged within and/or adjacent to the mixture volume (16, 17) and whose at least one whey electrode (18) is arranged within and/or adjacent to the calibration volume (20, 17).

## Revendications

1. Dispositif de mesure pour mesurer la proportion de caillé dans un mélange de caillé et de petit-lait, qui est reçu dans un récipient (16), le récipient est réalisé en particulier sous la forme d'un tube ou d'une cuve traversé(e) par un écoulement, dans lequel au moins une électrode pour mélange (14), d'un premier capteur, est agencée à l'intérieur d'un volume de mélange délimité par le récipient (16) et/ou dans la paroi du récipient (16), capteur qui est couplé à une unité d'évaluation pour déterminer une valeur de conductibilité,
**caractérisé en ce que**
au moins une électrode pour petit-lait (18), d'un second capteur, est agencée dans un volume de calibrage (20), qui contient sensiblement uniquement du petit-lait du mélange de caillé et de petit-lait, capteur qui est couplé à l'unité d'évaluation pour recalibrer de façon répétée le dispositif de mesure, dans lequel le volume de calibrage (20) comprend une unité de séparation, en particulier un filtre (26), un tamis et/ou une crépine, au moyen de laquelle le petit-lait est susceptible d'être séparé depuis le mélange de caillé et de petit-lait.

2. Dispositif de mesure selon la revendication 1,
**caractérisé en ce que** le volume de calibrage (20) est agencé dans un récipient à petit-lait séparé (20), qui est réalisé en particulier sous la forme d'un tube ou d'une cuve traversé(e) par un écoulement.

3. Dispositif de mesure selon la revendication 1,
**caractérisé en ce que** le récipient (16) tout comme le récipient à petit-lait (20) sont réalisés par un unique tube (17) traversé par un écoulement ou par une unique cuve traversée par un écoulement.

4. Dispositif de mesure selon l'une des revendications précédentes,
**caractérisé en ce que** les électrodes pour mélange (14) et/ou les électrodes pour petit-lait (18) sont agencées dans ou sur la paroi du tube (16, 17, 20) ou de la cuve, et de préférence le long d'une ligne enveloppe du tube (16, 20) ou de la cuve, et en particulier de manière également répartie.

5. Dispositif de mesure selon l'une des revendications précédentes,
**caractérisé en ce que** les électrodes pour mélange (14) et/ou les électrodes pour petit-lait (18) se terminent en affleurement avec la paroi intérieure du tube (16, 17, 20) ou de la cuve.

6. Dispositif de mesure selon l'une au moins des revendications précédentes,
**caractérisé en ce que** le récipient (16) et le récipient pour petit-lait (20) sont réalisés comme des tubes ou des cuves séparé(e)s, dont les zones d'entrée (22) sont reliées, en particulier via un élément partiel commun, à un réservoir (10) et/ou à un coagulateur pour le mélange de caillé et de petit-lait.

7. Dispositif de mesure selon la revendication 6,
**caractérisé en ce que** la zone de sortie (24) du récipient (16) et/ou la zone de sortie (24) du récipient à petit-lait (20) est/sont reliée(s) au réservoir (10) et/ou au coagulateur pour le mélange de caillé et de petit-lait.

8. Dispositif de mesure selon la revendication 6,
**caractérisé en ce que** la zone de sortie (24) du récipient (16) et/ou la zone de sortie (24) du récipient à petit-lait (20) est/sont reliée(s) à un conduit de convoyage (12), en particulier via un élément partiel commun, conduit qui mène en particulier à une unité de remplissage pour l'introduction du mélange de caillé et de petit-lait dans des moules à fromages.

9. Dispositif de mesure selon l'une au moins des revendications 2 à 5,
**caractérisé en ce que** la zone d'entrée (22) et la zone de sortie (24) du récipient à petit-lait (20) sont reliées au récipient (16), réalisé en particulier sous forme de réservoir (10) ou de coagulateur, pour le mélange de caillé et de petit-lait.

10. Dispositif de mesure selon l'une des revendications précédentes, **caractérisé en ce que** le volume de calibrage (20) est agencé à l'intérieur du récipient (16), réalisé en particulier sous forme de réservoir (10) ou de coagulateur, pour le mélange de caillé et de petit-lait.

11. Dispositif de mesure selon l'une des revendications précédentes,
**caractérisé en ce que** l'unité de séparation (26) comprend un tamis, en particulier un tamis à tube fendu, dont la surface définit un angle de 45° au maximum avec la direction d'écoulement.

12. Dispositif de mesure selon l'une au moins des revendications précédentes,
**caractérisé en ce que** l'unité d'évaluation est réalisée de telle façon que le recalibrage a lieu de façon retardée dans le temps, en particulier lorsqu'on atteint un équilibre dans le volume de mélange (16, 17) et/ou dans le volume de calibrage (20, 17).

13. Dispositif de mesure selon l'une au moins des revendications précédentes,
**caractérisé en ce que** l'un au moins des capteurs est réalisé sous forme de capteur de tomographie, en particulier de capteur dit "ERT".

14. Dispositif de mesure selon l'une au moins des revendications précédentes,
**caractérisé en ce que** l'un au moins des capteurs est réalisé additionnellement pour la mesure de température et/ou de pression et/ou de volume.

15. Dispositif de mesure selon l'une au moins des revendications précédentes,
**caractérisé en ce que** le premier et le second capteur sont réalisés sous forme de capteur commun, dans lequel ladite au moins une électrode pour mélange (14) est agencée à l'intérieur du volume pour mélange (16, 17) et/ou de manière adjacente à celui-ci, et dans lequel ladite au moins une électrode pour petit-lait (18) est agencée à l'intérieur du volume de calibrage (20, 17) et/ou de manière adjacente à celui-ci.
